(19) Europäisches Patentamt
European Patent Office
Office européen des brevets

(11) **EP 3 678 065 A1**

(12) **EUROPEAN PATENT APPLICATION**
published in accordance with Art. 153(4) EPC

(43) Date of publication:
**08.07.2020 Bulletin 2020/28**

(51) Int Cl.:
***G06N 5/02*** *(2006.01)*

(21) Application number: **18851869.0**

(22) Date of filing: **08.08.2018**

(86) International application number:
**PCT/CN2018/099310**

(87) International publication number:
**WO 2019/042099 (07.03.2019 Gazette 2019/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.08.2017 CN 201710770922**

(71) Applicants:
• **Jiangsu Kanion Pharmaceutical Co., Ltd.
Jiangsu 222047 (CN)**
• **Zhejiang University
Hangzhou, Zhejiang 310058 (CN)**

(72) Inventors:
• **XIAO, Wei
Lianyungang
Jiangsu 222047 (CN)**
• **LIU, Xuesong
Hangzhou
Zhejiang 310058 (CN)**
• **LING, Ya
Lianyungang
Jiangsu 222047 (CN)**
• **CHEN, Yong
Hangzhou
Zhejiang 310058 (CN)**

• **WANG, Zhenzhong
Lianyungang
Jiangsu 222047 (CN)**
• **JIANG, Xiaohong
Hangzhou
Zhejiang 310058 (CN)**
• **LI, Yerui
Hangzhou
Zhejiang 310058 (CN)**
• **BAO, Lewei
Lianyungang
Jiangsu 222047 (CN)**
• **ZHANG, Chenfeng
Lianyungang
Jiangsu 222047 (CN)**
• **WANG, Lei
Hangzhou
Zhejiang 310058 (CN)**
• **CHEN, Yongjie
Lianyungang
Jiangsu 222047 (CN)**

(74) Representative: **Kurig, Thomas
Becker & Kurig Partnerschaft
Patentanwälte PartmbB
Bavariastrasse 7
80336 München (DE)**

(54) **CHINESE MEDICINE PRODUCTION PROCESS KNOWLEDGE SYSTEM**

(57) Disclosed is a process knowledge system for traditional Chinese medicine production. The system comprises a database module comprising a production data acquisition unit and a storage unit, wherein the production data acquisition unit is used for acquiring process parameter data in production, the parameter data comprises quality data and process data, and the storage unit is used for storing the acquired process parameter data; a capability evaluation module used for evaluating the process capability of the system according to the quality data to obtain a process capability evaluation result; a monitoring feedback module used for entering a whole-process monitoring mode in response to the process capability evaluation result indicating that the process capability is sufficient; and a design space searching module used for entering a design space searching mode in response to the process capability evaluation result indicating that the process capability is insufficient. According to the present invention, release parameters are determined or a design space is searched for through process capability evaluation, so that a production process knowledge system stepwise regresses into a knowledge process system capable of realizing intelligent regulation and feedback of the traditional Chinese medicine production process.

**(Cont. next page)**

FIG. 8

**Description**

Technical Field

The present invention relates to the field of process knowledge systems, in particular to a process knowledge system for traditional Chinese medicine production.

Background

Under the production control of a process knowledge system (abbreviated as PKS), improvements against traditional production line techniques are principally reflected in the following aspects: (1) the new workflow is an integral system, while each work station on a traditional production line is relatively independent and works independently, the correlation among all work stations is weak, and the degree of concentration is low; the new production process is an integral system, and all steps are mutually correlated and influenced, so that overall optimization of the production process is realized; (2) the production process of the PKS is more informationalized and automated, while in the traditional production line, each independent link should be monitored by special workers, information is recorded by special workers, the working efficiency is closely related to the condition of workers, and errors may be caused by negligence of the workers; the new workflow is fully automated and intelligent, all procedures are controlled by the informationalized system, data acquisition, storage and extraction are informationalized, parameter setting is automated, and quality detection is intelligent, so that the efficiency is improved to a great extent, and errors caused by negligence of workers are reduced; and (3) the PKS technique can feed back and adjust production control parameters in real time, thus being able to control product quality more stably.

However, existing PKSs have simple functions and still have many drawbacks in knowledge mining, thus falling far short of the intelligent production management level and needing to be improved in the aspect of control accuracy of process parameters.

Summary

In order to solve the problems of the prior art, the present invention provides a process knowledge system for traditional Chinese medicine production. The technical solution is as follows:

In one aspect, the present invention provides a process control method for traditional Chinese medicine production. The process control method for traditional Chinese medicine production comprises:

Acquiring process parameter data in production, wherein the process parameter data comprises quality data and process data;

Evaluating the process capability of a system according to the quality data to obtain a process capability evaluation result;

Entering a whole-process monitoring mode if the process capability evaluation result indicates that the process capability is sufficient; or

Entering a design space searching mode according to the process data if the process capability evaluation result indicates that the process capability is insufficient.

Furthermore, the step of entering a design space searching mode comprises:

Acquiring the process data, wherein the process data comprises quality parameters of an intermediate obtained in a previous work section;

Selecting a type of a critical quality attribute according to work section production conditions;

Screening out process data related to the critical quality attribute, and using the screened-out process data as a critical process parameter;

Establishing a relationship model between the critical process parameter and the critical quality attribute; and

Acquiring a design space according to the relationship model, wherein the design space is a specific range corresponding to the critical quality attribute.

Furthermore, the process control method for traditional Chinese medicine production further comprises the following steps to be performed after the step of entering a design space searching mode:

Releasing parameters according to the acquired design space;

Re-evaluating the process capability of the system to obtain a process capability re-evaluation result;

Entering the whole-process monitoring mode if the process capability re-evaluation result indicates that the process capability is sufficient; or

Mining potential parameters of the design space if the process capability re-evaluation result indicates that the process capability is insufficient.

Furthermore, the step of mining potential parameters of the design space comprises:

Receiving a request for mining potential parameters of the design space, wherein the request comprises work section condition information corresponding to the design space;

Acquiring a critical quality attribute corresponding to the work section according to the work section condition information and determining a potential parameter set;

Testing determined potential parameters to obtain to-be-verified potential parameters; and

Verifying the to-be-verified potential parameters to obtain the potential parameters of the design space.

Furthermore, the step of evaluating the process capability of a system to obtain a process capability evaluation result comprises:

Acquiring quality data to obtain a quality sample, wherein the quality data are performance parameters of the intermediate in the production process;

Obtaining a process average value and a process standard deviation according to the quality sample;

Carrying out data screening on the quality sample to obtain a quality control standard sample;

Obtaining a quality control standard upper limit and/or lower limit according to the quality control standard sample;

Obtaining a standard median value and a process dispersion value according to the quality control standard upper limit and lower limit, wherein,

$$\text{standard median value} = \frac{\text{upper limit} + \text{lower limit}}{2}$$

$$\text{process dispersion value} = \frac{\text{upper limit} - \text{lower limit}}{2};$$

Obtaining a quality evaluation value according to the standard median value, the process dispersion value, the process average value and the process

$$\text{quality evaluation value} = \frac{\text{process dispersion value} - |\text{process average value} - \text{standard median value}|}{3 * \text{process standard deviation}}$$

standard deviation by the following calculation formula;
, wherein, the process average value is an average of quality data of the quality sample, and the process standard deviation is a standard deviation of the quality data; and

Obtaining the process capability evaluation result according to a quality evaluation result.

Furthermore, the step of entering the whole-process monitoring mode comprises multi-parameter recognition, which comprises:

Acquiring multiple training samples to form a training sample set, wherein each training sample comprises multiple process parameters, each process parameter has a corresponding attribute parameter and a corresponding class, and there are multiple combinations of the attribute parameters and classes;
Acquiring a distribution transmission information value of the training sample set according to the classes in the training sample set;
Acquiring an information gain of each process parameter according to the distribution transmission information value;
Selecting the process parameter with the maximum information gain as a split node to establish a decision tree; and
Carrying out class recognition on new data according to the decision tree.

Furthermore, the step of entering the whole-process monitoring mode comprises process parameter-based result feedback which comprises:

Receiving a result feedback request, wherein the result feedback request comprises an intermediate result type;

Acquiring process parameters corresponding to the feedback request to form a process parameter set, wherein the process parameters are multi-dimensional parameters; and

Inputting the process parameter set to a result feedback neural network model, wherein the result feedback neural network model is obtained by training with process parameter samples; and

Acquiring an output result of the result feedback neural network model.

Furthermore, the step of obtaining the result feedback neural network model by training with the process parameter samples comprises:

Acquiring data of process parameter samples to be trained, wherein the process parameter samples comprise multiple process parameter sets and corresponding given target values;

Establishing an initial network model, wherein the initial network model comprises an input layer, a hidden layer, an output layer, an initial weight and an initial offset; and

Updating the initial weight and the initial offset through a back-propagation method until weight convergence is realized, so as to obtain the result feedback neural network model.

In the other aspect, the present invention provides a process knowledge system for traditional Chinese medicine production. The process knowledge system for traditional Chinese medicine production comprises:

A database module comprising a production data acquisition unit and a storage unit, wherein the production data acquisition unit is used for acquiring process parameter data in production, parameter data comprises quality data and process data, and the storage unit is used for storing the acquired process parameter data;

A capability evaluation module used for evaluating the process capability of a system according to the quality data to obtain a process capability evaluation result;

A monitoring feedback module used for entering a whole-process monitoring mode in response to the process capability evaluation result indicating that the process capability is sufficient; and

A design space searching module used for entering a design space searching mode in response to the process capability evaluation result indicating that the process capability is insufficient.

Furthermore, the design space searching module comprises:

A process data unit used for acquiring the process data, wherein the process data comprises quality parameters of an intermediate obtained in a previous work section;

A CQA unit used for selecting a type of a critical quality attribute according to work section production conditions;

A CPP unit used for screening out process data related to the critical quality attribute so as to use the screened-out process data as a critical process parameter;

A design space model unit used for establishing a relationship model between the critical process parameter and the critical quality attribute; and

A space unit used for acquiring a design space according to the relationship model, wherein the design space is a specific range corresponding to the critical quality attribute.

Furthermore, the process knowledge system for traditional Chinese medicine production further comprises a mining module which comprises:

A mining request unit used for receiving a request for mining potential parameters of the design space, wherein the request comprises work section condition information corresponding to the design space;

A determining unit used for acquiring the critical quality attribute corresponding to a work section according to the work section condition information and determining a potential parameter set;

A mining execution unit used for testing determined potential parameters to obtain to-be-verified potential parameters; and

A verification unit used for verifying the to-be-verified potential parameters to obtain the potential parameters of the design space.

Furthermore, the capability evaluation module comprises:

A data acquisition unit used for acquiring quality data to obtain a quality sample, wherein the quality data is performance parameters of the intermediate in the production process;
A process processing unit used for obtaining a process average value and a process standard deviation according to the quality sample;
A screening unit used for carrying out data screening on the quality sample to obtain a quality control standard sample;
A standard range unit used for obtaining a quality control standard upper limit and/or lower limit according to the quality control standard sample;
An evaluation value unit used for obtaining a standard median value and a process dispersion value according to the quality control standard upper limit and lower limit, wherein,

$$\text{standard median value} = \frac{\text{upper limit} + \text{lower limit}}{2}$$

$$, \text{process dispersion value} = \frac{\text{upper limit} - \text{lower limit}}{2};$$

and
Obtaining a quality evaluation value according to the standard median value, the process dispersion value, the process average value and the process standard deviation by the following calculation formula;

$$\text{quality evaluation value} = \frac{\text{process dispersion value} - |\text{process average value} - \text{standard median value}|}{3 \cdot \text{process standard deviation}}$$

, wherein, the process average value is an average of quality data of the quality sample, and the process standard deviation is a standard deviation of the quality data; and

A mapping unit used for obtaining the process capability evaluation result according to the quality evaluation result.

Furthermore, the monitoring feedback module is used for multi-dimensional parameter recognition and comprises:

A training sample acquisition unit used for acquiring multiple training samples to form a training sample set, wherein each training sample comprises multiple process parameters, each process parameter has a corresponding attribute parameter and a corresponding class, and there are multiple combinations of the attribute parameters and classes;

A distribution transmission unit used for acquiring a distribution transmission information value of the training sample set according to the classes in the training sample set;

A gain unit used for acquiring an information gain of each process parameter according to the distribution transmission information value;

A decision tree unit used for selecting the process parameter with the maximum information gain as a split node to establish a decision tree; and

A data recognition unit used for carrying out class recognition on new data according to the decision tree.

Furthermore, the monitoring feedback module is used for carrying out result feedback based on process parameters and comprises:

A result feedback request unit used for receiving a result feedback request which comprises an intermediate result type;

A parameter unit used for acquiring process parameters corresponding to the feedback request to form a process parameter set, wherein the process parameters are multi-dimensional parameters; and

A result feedback neural network model input unit used for inputting the process parameter set to a result feedback neural network model which is obtained by training process parameter samples; and

A result feedback neural network model output unit used for acquiring an output result of the result feedback neural network model.

Furthermore, the monitoring feedback module further comprises a result feedback neural network model training unit which comprises:

A training sample acquisition unit used for acquiring data of process parameter samples to be trained, wherein the process parameter samples comprise multiple process parameter sets and corresponding given target values;

An initial model unit used for establishing an initial network model which comprises an input layer, a hidden layer, an output layer, an initial weight and an initial offset; and

A weight updating unit used for updating the initial weight and the initial offset through a back-propagation method until weight convergence is realized, so as to obtain the result feedback neural network model.

The technical solution provided by the present invention has the following beneficial effects:

1) The process capability in production is digitized and graded to be as clear as possible, so that a guidance is provided for process control;

2) The critical process parameter relating to the critical quality attribute is screened out through RSD analysis and correlation analysis to provide reliable parameter data for model establishment; and the relationship model between the critical process parameter and the critical quality attribute is established through a stepwise regression method, so that the influence of multicollinearity is avoided, and the reliability of the model is high;

3) A single-factor test is carried out on potential parameters to obtain multiple process parameters, so that a foundation is laid for potential parameter mining; and an orthogonal test is carried out on the potential parameters, and to-be-verified potential parameters relating to the critical quality attribute are screened out through variance analysis, so that reliable parameter data is provided for reconstruction of the design space;

4) The information gain is used as the selection standard of the split node of the decision tree, so that the accuracy of the decision tree is improved; and the decision tree model is established to realize accurate classification of multi-dimensional data; and

5) The weight and offset of the neural network model are updated through the back-propagation method, so that the classification accuracy of the model is improved constantly; and input parameters in the PKS production process are classified through the result feedback neural network model to check whether or not reactants are qualified, so that intelligent feedback is realized.

Brief Description of the Drawings

For the sake of a clearer explanation of the technical solutions of the embodiments of the present invention, a brief description of the drawings used for describing the embodiments is given below. Clearly, the drawings in the following description are only for illustrating some embodiments of the present invention, and those ordinarily skilled in the art can obtain other drawings according to the following ones without creative labor.

FIG. 1 is a flow diagram of a process control method for traditional Chinese medicine production in one embodiment of the present invention;

FIG. 2 is a simplified diagram of a whole-process production control method in one embodiment of the present invention;

FIG. 3 is a flow diagram of the whole-process production control method in the embodiment of the present invention;

FIG. 4 is a flow diagram of a method for training a result feedback neural network model in one embodiment of the present invention;

FIG. 5 is a flow diagram of a method for searching for a design space in one embodiment of the present invention;

FIG. 6 is a flow diagram of a method for mining potential parameters of the design space in one embodiment of the present invention;

FIG. 7 is a flow diagram of a method for evaluating the process capability of a system in one embodiment of the present invention;

FIG. 8 is a block diagram of modules of a process knowledge system for traditional Chinese medicine production in one embodiment of the present invention;

FIG. 9 is a structural diagram of a decision tree in one embodiment of the present invention;

FIG. 10 is a schematic diagram of a multi-layer feedforward neural topological structure in one embodiment of the present invention; and

FIG. 11 is a schematic diagram of an input-output structure of one neural network cell in one embodiment of the present invention.

Detailed Description of Embodiments

In order to make those skilled in the art have a better understanding of the solutions of the present invention, the technical solutions of the embodiments of the present invention are clearly and completely described below in combination with the accompanying drawings of the embodiments. Clearly, the embodiments in the following description are only illustrative ones, and are not all possible ones of the present invention. All other embodiments obtained by those ordinarily skilled in the art on the basis of the following ones without creative labor should also fall within the protection scope of the present invention.

Embodiment 1

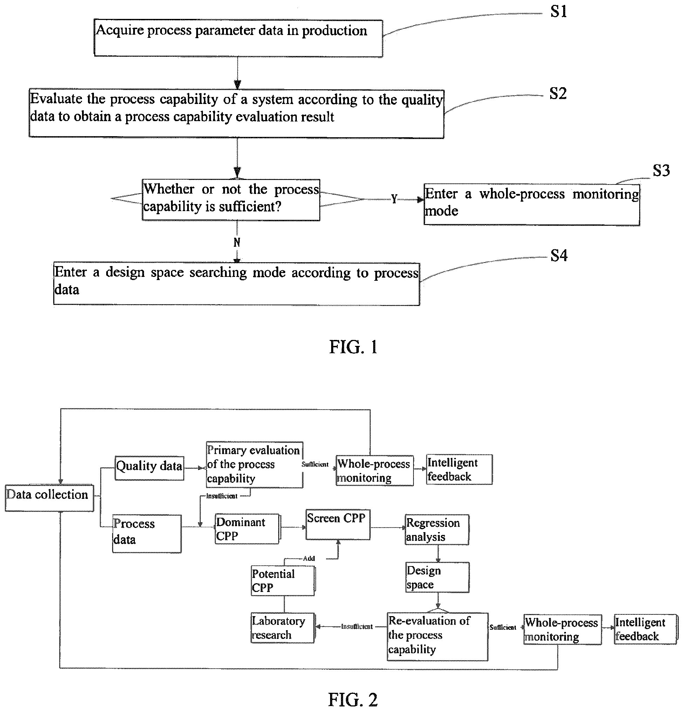

In one embodiment of the present invention, a process control method for traditional Chinese medicine production is provided. Referring to FIG. 1, the method comprises the following steps:

S1: process parameter data in production is acquired, wherein the process parameter data comprises quality data and process data.
Particularly, the quality data is used as a data basis for evaluating the process capability of a system, and the process data is used as a data basis for assisting in searching for a design space, as shown in FIG. 2.
Wherein, the quality data are performance parameters of an intermediate in the production process and particularly comprise the content, transfer rate, purity, and the like, of index components of the intermediate.
S2: the process capability of the system is evaluated according to the quality data to obtain a process capability evaluation result; if the process capability evaluation result indicates that the process capability is sufficient, S3 is performed; otherwise, S4 is performed.
Particularly, quality data samples are processed to obtain a quality evaluation result, and a specific processing method will be detailed below in Embodiment 5.
S3: entering a whole-process monitoring mode.
If the process capability evaluation result shows that the production capacity of the work section meets production requirements, it indicates that the quality stability and batch consistency of intermediates are higher under the current process parameters of the work section, the production capacity of the work section is good, in this case, normally-distributed $2\sigma$ or $3\sigma$ of the current process parameters can be selected to serve as a process parameter release standard; a control chart is established through an MSPC method to monitor the whole production process and the endpoint of the production process; a real-time trend display module of the PKS is embedded to realize real-time monitoring and online warning of the whole production process.
A PKS platform automatically collects batch data in one cycle every constant cycles to automatically evaluate the process capability, so that changes of the process capability of each work section in production can be continuously acquired, and the parameter release range of each work section can be continuously updated; and the release range is updated on the real-time trend display module, so that whole-process monitoring and online warning are realized.
S4: enter a design space searching mode according to the process data.

If the process capability is insufficient, it indicates that the current production parameters cannot meet product production requirements and need to be adjusted, that is a design space needs to be searched for to search out optimized release conditions for the production system which has an insufficient capability currently.

Embodiment 2

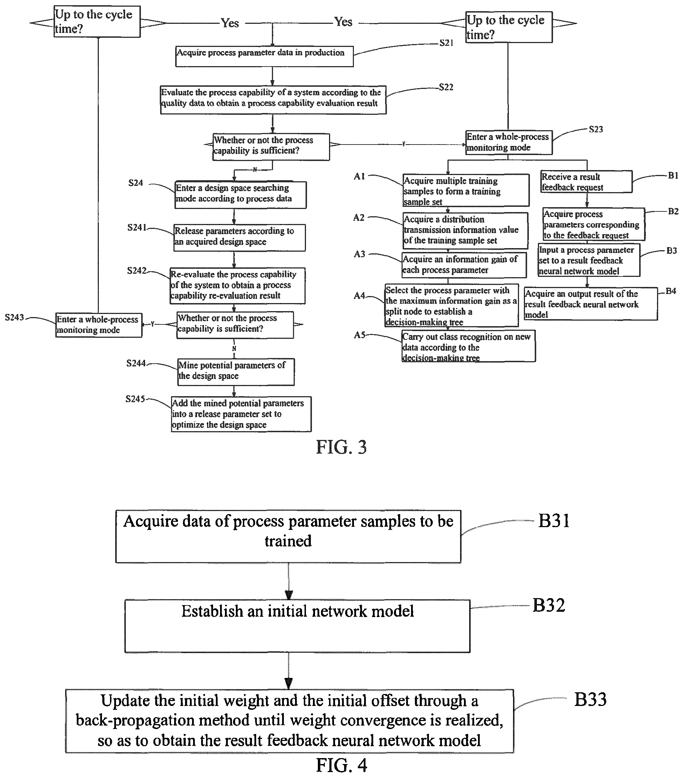

In one embodiment of the present invention, a complete production control method for a traditional Chinese medicine production process is provided. Referring to FIG. 2 and FIG. 3, the method comprises the following steps:

S21: process parameter data in production is acquired, wherein the process parameter data comprises quality data and process data; and

S22: the process capability of a system is evaluated according to the quality data to obtain a process capability evaluation result; if the process capability evaluation result indicates that the process capability is sufficient, S23 is performed; otherwise, S24 is performed, as mentioned in Embodiment 1.

S23: enter a whole-process monitoring mode.

In one aspect, as mentioned in Embodiment 1, after entering the whole-process monitoring mode, real-time monitoring can be realized through the following steps:

A1: multiple training samples are acquired to form a training sample set;

A2: a distribution transmission information value of the training sample set is acquired according to classes in the training sample set;

A3: an information gain of each process parameter is acquired according to the distribution transmission information value;

A4: the process parameter with the maximum information gain is selected as a split node to establish a decision tree; and

A5: class recognition is carried out on new data according to the decision tree.

Wherein, the distribution transmission information value is obtained by the following calculation formula: info $(S) = -\sum Pi*\log_2(Pi)$, wherein info(S) is the distribution transmission information value of the training sample set, and $Pi$ is the probability of the $i^{th}$ class of the training samples.

For example, as shown in Table 1:

Table 1

| Serial number | T | P | PH | Class |
|---|---|---|---|---|
| 1 | H | N | H | good |
| 2 | L | H | N | good |
| 3 | N | L | L | excellent |
| 4 | H | N | N | poor |
| 5 | N | N | H | good |
| 6 | L | N | N | excellent |
| 7 | L | H | L | excellent |
| 8 | N | H | H | poor |
| 9 | H | L | N | good |

The training sample set comprises nine training samples, wherein three training samples (serial numbers 3, 6 and 7) are classified as excellent, four training samples are classified as good, and two training samples are classified as poor.

$$\text{info}\ (S) = -\frac{3}{9}*\log_2\frac{3}{9} - \frac{4}{9}*\log_2\frac{4}{9} - \frac{2}{9}*\log_2\frac{2}{9} = 1.0683$$

The information gain is obtained by the following calculation formula:

$$gain\ (S, A) = \text{info}(S) - \sum_{v \in Varies(A)} \frac{|S_v|}{|S|}*\text{info}(S_v)$$

, wherein info(S) is the distribution transmission information value of the training sample set, info($S_v$) is a distribution transmission information value of an attribute parameter, $\dfrac{|S_v|}{|S|}$ is the probability of the attribute parameter in a class, and $Varies(A)$ is an attribute parameter set.

Take data in Table 1 as an example,

$$gain(S,T) = 1.0683 - [\frac{3}{9} \times \text{info}(T_H) + \frac{3}{9} \times \text{info}(T_N)\frac{3}{9} \times \text{info}(T_L)]$$

, as for info($T_H$),

$$\text{info}(T_H) = -(\frac{0}{3}*\log_2\frac{0}{3} + \frac{2}{3}*\log_2\frac{2}{3} + \frac{1}{3}*\log_2\frac{1}{3}) = 0.9183$$

; similarly, info($T_N$) and info($T_L$) can be obtained, then gain(S,T) is obtained; and $gain(S,P)$ and $gain(S,PH)$ are obtained in a similar way.

If $gain(S,T)_{>gain(S,PH)>gain(S,P)}$ by calculation, a model is established with temperature T as a split node.

In the embodiment of the present invention, the information gain is used as a criterion for selecting the split node for establishing the decision tree, so that accurate recognition and classification of two-dimensional (multi-dimensional) data are facilitated, and a reliable basis is provided for intelligent feedback of parameters. The two-dimensional (multi-dimensional) data can be applied in the following scenes: the process parameter in one training sample is a critical process parameter obtained by screening according to the critical quality attribute, the critical quality attribute is an attribute parameter selected according to work sections in the process knowledge system, and one critical quality attribute has two or more critical process parameters. In these scenes, the type of parameters can be recognized through the method in the embodiment of the present invention.

Use the process parameter temperature $T$ having the maximum information gain as a root node, and the attribute parameter corresponding to the process parameter temperature $T$ as a first branch node, whether the classes corresponding to the attribute parameters are consistent or not is judged; if yes, the class is used as a leaf node; otherwise, this step is repeated with the process parameter with the secondary maximum information gain as a second branch node until the class used as the leaf node is obtained.

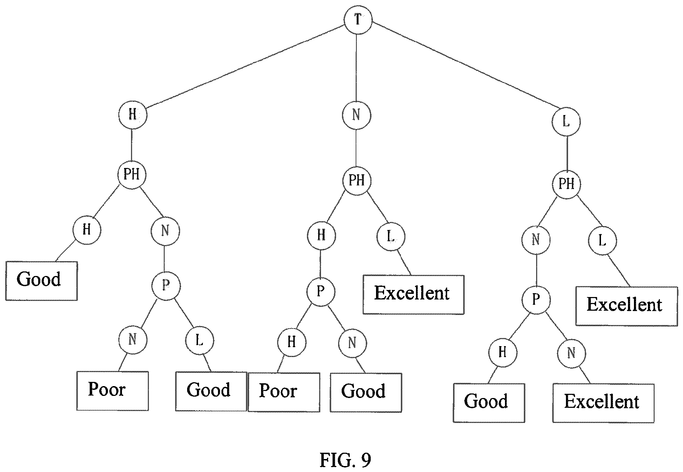

With data in Table 1 as an example, if it is obtained by calculation that $gain(S,T)>gain(S,PH)>gain(S,P)$, a decision tree shown in FIG. 9 is established.

According to the decision tree shown in FIG. 9, the type of new data can be judged. For example, as for new data T(L) P(H) PH(L), although the new data is not shown in Table 1, this parameter can be recognized as excellent according to the decision tree shown in FIG. 9. For another example, new data T(N) P(H) PH(H) can be judged as poor and is fed back to users in time, or the users are reminded and warned.

If the root nodes and every branch nodes are the same and the leaf nodes are different, leaf nodes under the root nodes and the branched nodes in the training sample set are counted; if the numbers of the leaf nodes are consistent, any one of the leaf nodes is abandoned randomly; or, the leaf node having a small number is abandoned. For example, a training sample T(N) P(H) PH(H) numbered 10 and classified as good is added in Table 1, clearly, the class of this training sample is different from that of sample numbered 8 in Table 1, in this case, numbers of two samples are counted, and the class obtained on the basis of more samples prevails; if the numbers of samples in the two cases are identical, any one class is selected randomly.

In the other aspect, as mentioned in Embodiment 1, after the system enters the whole-process monitoring mode, online warning can be realized through the following steps:

B1: a result feedback request is received, wherein the result feedback request comprises an intermediate result type;

B2: process parameters corresponding to the feedback request are acquired to form a process parameter set, wherein the process parameters are multi-dimensional parameters; and

B3: the process parameter set is input to a result feedback neural network model, wherein:
The result feedback neural network model is obtained by training process parameter samples through the following steps, as shown in FIG. 4:

B31: data of process parameter samples to be trained is acquired, wherein the process parameter samples comprise multiple process parameter sets and corresponding given target values;

B32: an initial network model is established, wherein the initial network model comprises an input layer, a hidden layer, an output layer, an initial weight and an initial offset; and

B33: the initial weight and the initial offset are updated through a back-propagation method until weight convergence is realized, so as to obtain the result feedback neural network model;

B4: an output result of the result feedback neural network model is acquired.

In an error back-propagation process, errors are shared by all cells of all layers, so that error signals of all the cells of all the layers are obtained to amend weights of all the cells. That is, the error back-propagation process is a weight adjustment process.

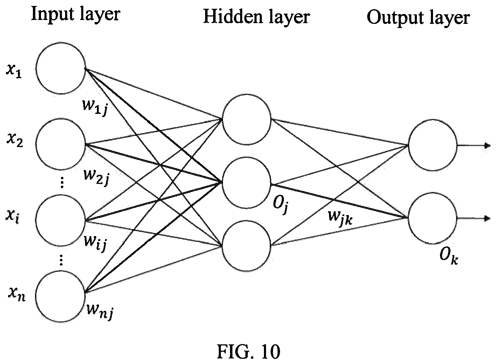

Referring to FIG. 10 which is a schematic diagram of a topological structure of a multi-layer feedforward neural network, the multi-layer feedforward neural network comprises an input layer, a hidden layer and an output layer. The input layer comprises a plurality of cells i, the hidden layer comprises a plurality of cells j, and the output layer comprises a plurality of cells k. As shown in FIG. 10, the connection weight of the cells i of the input layer and the cells j of the hidden layer is $w_{ij}$, and the connection weight of the cells j of the hidden layer and the cells k of the output layer is $w_{jk}$.

In another embodiment of the present invention, the neural network model comprises more than one hidden layer(not shown in FIG. 10). The model establishing method, the weight and offset updating method, of the neural network model comprising more than one hidden layer are the same as those of the neural network model comprising one hidden layer. The neural network model comprising one hidden layer is explained below as an example:

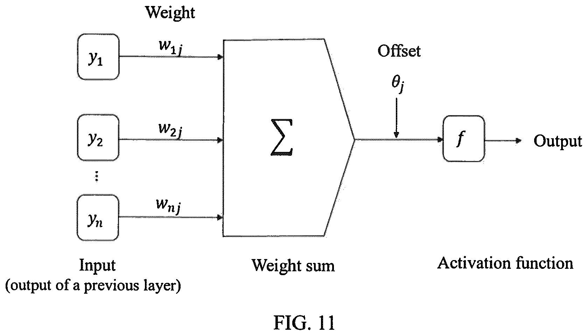

The weight of the neural network is initialized into a small random number, and each neural network cell (abbreviated to network cell) has an associated offset which is also initialized into a small random number. Referring to FIG. 11, with one neutral cell j in the hidden layer as an example:

The calculation formula of a net input of the cell j is as follows:

$$I_j = \sum_{i=1}^{n1} O_i w_{ij} + \theta_j$$

, wherein the cell j of this layer has n1 cells connected with a previous layer, $O_i$ is an output value of the cell i of the previous layer, $w_{ij}$ is the connection weight of the cell i of the previous layer and the cell j of this layer, $\theta_j$ is an offset of the cell j of this layer and serves as a threshold to change the activity of the nerve cell.

The calculation formula of an output of the cell j is as follows:

$$O_j = \frac{1}{1+e^{-I_j}}$$

, wherein $O_j$ is an actual output of the cell j of this layer.

The process of calculating back-propagation errors comprises error correction of the initial weight and the initial offset, so that the weight and the offset are updated.

In one embodiment of the present invention, a weight error coefficient is obtained by the following formula: $Err_j = O_j(1-O_j)(T_j-O_j)$, wherein $T_j$ is a given target value of a given training cell block of the cell j, $O_j$ is the actual output of the cell j of this layer, and $O_j(1-O_j)$ is a derivative of a Logistic function.

In another embodiment of the present invention, the weight error coefficient is different according to the output layer or the hidden layer. In this embodiment, the weight error coefficient of the output layer and the weight error coefficient of the hidden layer are respectively defined, wherein the weight error coefficient of the cell j of the output layer (namely the weight error coefficient of the output layer) is obtained by the following formula:

$Err_j = O_j(1-O_j)(T_j-O_j)$, wherein $T_j$ is the given target value of the given training cell block of the cell j, and $O_j$ is the actual output of the cell j of this layer;

The weight error coefficient of the cell j of the hidden layer (namely the weight error coefficient of the hidden layer) is obtained by the following formula:

$$Err_j = O_j(1-O_j)\sum_{k=1}^{n2} Err_k w_{jk}$$

, wherein the cell j of this layer has n2 cells connected with a next layer, $Err_k$ is the weight error coefficient of the cell k of the next layer, $w_{jk}$ is the connection weight of the cell j of this layer and the cell k of the next layer, $O_j$ is an actual output of the cell j of this layer, and $O_j(1-O_j)$ is the derivative of the Logistic function.

On this basis, the initial weight is updated as follows:

The weight error coefficient is acquired to obtain a weight variation $\Delta w_{ij} = l(Err_j O_i)$; and

The weight $w_{ij} = w_{ij} + \Delta w_{ij}$ is updated according to the weight variation, wherein $w_{ij}$ is the connection weight of the cell i of the previous layer and the cell j of this layer, $\Delta w_{ij}$ is the variation of the connection weight of the cell i of the previous layer and the cell j of this layer, $Err_j$ is the weight error coefficient of the cell j, $O_i$ is the output of the cell i of the previous layer, and $l$ is a learning rate which is typically a constant value within the range of (0.0, 1.0).

In the other aspect, the initial offset is updated as follows:

The weight error coefficient is acquired to obtain an offset variation $\Delta\theta_j = (-l)Err_j$; and

The offset $\theta_j = \theta_j + \Delta\theta_j$ is updated according to the offset variation, wherein $\theta_j$ is the offset of the cell j of this layer, $\Delta\theta_j$ is the offset variation, $Err_j$ is the weight error coefficient of the cell j, $l$ is the learning rate which is typically a constant value within the range of (0.0, 1.0), the learning rate for weight updating and the learning rate for offset updating can be independently set (as different values) or set as the same value which is preferably 0.3.

Weight and offset update carried out every time a sample is processed, called case update. Weight and offset update carried out after all cell blocks in the training set are processed, called epoch update. Theoretically, epoch update is adopted for data derivation of the back-propagation algorithm; however, in practice, it is through case update that more accurate results can be obtained.

Furthermore, the weight convergence meets any one of the following conditions:

The variation of weight update is smaller than a preset first threshold, that is, $\Delta w_{ij}$ is smaller than a specified threshold;

The percentage of misclassified cell blocks of the result feedback neural network model is smaller than a preset second threshold, for example, a sample is classified by means of the established model, and the misclassification ratio of the model is less than 5% with reference to a given target value; and

The times of weight update reach a preset third threshold, for example, the times of epoch update reach 100,000.

Training is stopped if any one of the above conditions is met. In practice, the weight convergence can be achieved after hundreds of thousands of cycles. There are many experience and skills for training of the neural network, and the simulated annealing technique is preferably adopted to guarantee global optimum of the convergence of the neural network.

The result feedback neural network model is established by training and learning according to the above algorithm; every time a process parameter is input, the model provides a feedback result indicating whether or not the intermediate is qualified, that is, after the model outputs a result, the result is compared with a preset acceptability criterion to provide the feedback result indicating whether or not the intermediate is qualified (or excellent/good/medium/poor).

In another aspect, in the whole-process monitoring mode, the system platform automatically performs S21 repeatedly every constant cycles to update the control chart of whole-process monitoring, as shown in FIG. 2.

S24: the system enters a design space searching mode according to the process data;

S241: parameters are released according to the acquired design space;

S242: the process capability of the system is re-evaluating to obtain a process capability re-evaluation result; if the re-evaluation result indicates that the process capability is sufficient, S243 is performed; otherwise, S244 is performed;

S243: enter the whole-process monitoring mode.

Similarly, in the whole-process monitoring mode, the system platform automatically performs S21 repeatedly every constant cycles to update the control chart of whole-process monitoring.

S244: potential parameters of the design space are mined;

S245: the mined potential parameters are added into a release parameter set to optimize the design space until the process capability re-evaluation result indicates that the process capability is sufficient.

The method for mining the potential parameters of the design space will be specifically detailed below in Embodiment 4.

Embodiment 3

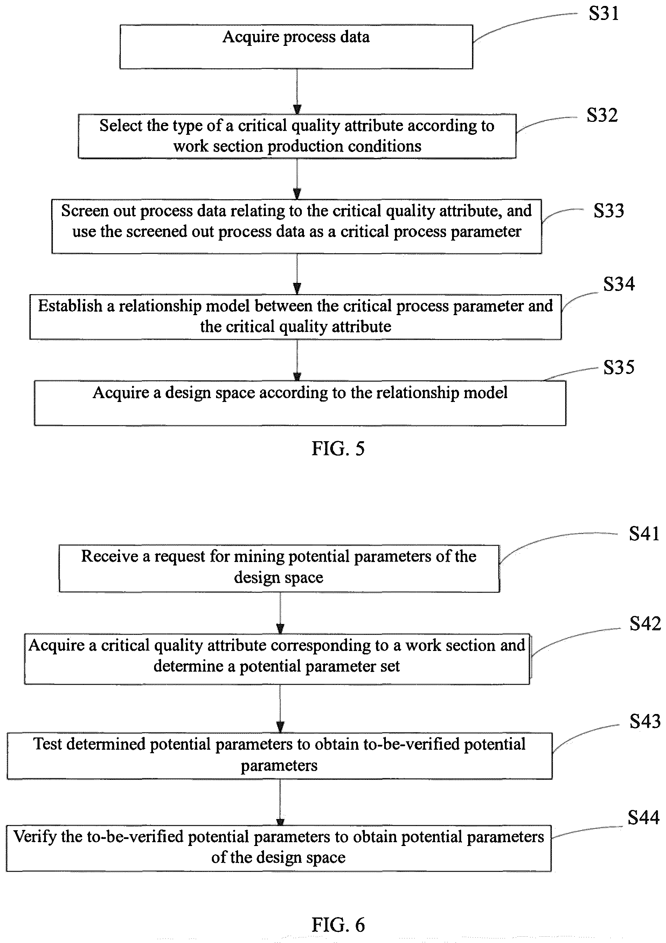

In one embodiment of the present invention, a method for searching for a design space is provided. Referring to FIG. 5, the method comprises the following steps:

S31: process data is acquired, wherein the process data comprises quality parameters of an intermediate obtained in a previous work section;

S32: the type of a critical quality attribute is selected according to work section production conditions;

S33: process data relating to the critical quality attribute is screened out and used as a critical process parameter;

S34: a relationship model between the critical process parameter and the critical quality attribute is established; and

S35: a design space is acquired according to the relationship model, wherein the design space is a specific range corresponding to the critical quality attribute.

Wherein, the critical process parameter is screened out as follows:
Because some process parameters are constant or some process parameters vary within a small range during actual production, not all process parameters have analysis value, and these slightly-varying parameters can be regarded as constants and will not be analyzed. In view of this, relative standard deviation analysis is carried out on the process parameters to obtain process parameters in accordance with a deviation threshold.

Herein, relative standard deviation (RSD) analysis is adopted for the purpose of obtaining drastically-fluctuating process parameters by primary screening, that is, the process parameters in accordance with the deviation threshold are obtained.

The calculation formula of RSD is as follows:

$$RSD = \sqrt{\frac{\sum (x_i - \bar{x})^2}{n-1}} \Big/ \frac{\sum x_i}{n}$$

, wherein $x_i$ is the value of a process parameter of each batch of samples, n is the sample capacity, and $\bar{x}$ is the average of the process parameter of a sample.

Generally, parameters with large RSD ($\geq 3\%$) are selected for correlation analysis, and as a reference, the threshold of RSD can be set as 3%. however, the actual threshold of RSD should be determined by repeated analysis.

In addition, correlation analysis is carried out on process parameters in accordance with the deviation threshold to obtain a correlation coefficient between the process parameters and the critical quality attribute.

In actual production, different process parameters have different degrees of influence on the quality of the intermediate, some parameters have decisive influence on the quality of a final product, while some parameters have slight influence on the quality of the intermediate or the final product, the critical process parameter having an influence on the product and the contribution degree of the critical process parameter can be visually seen through the correlation analysis, so as to find out the inherent law of the production process and to deepen the understanding of the production process, and then the production parameters can be better controlled in a targeted manner.

In the embodiment of the present invention, the correlation between CQA and CPP (or CPPs) is evaluated by means of the Pearson coefficient, having a value from -1 to 1, in statistics. The correlation coefficient of the two variables is mathematically defined as:

$$\rho_{X,Y} = \frac{cov\ (X,Y)}{\sigma_X \sigma_Y}$$

, wherein, $\sigma_X$ is the standard deviation of attribute X which is a characteristic relating to process parameters such as pressure average value, temperature average value, variance and time characteristic; $\sigma_Y$ is the standard deviation of attribute Y which is CQA such as index component transfer rate, total acid transfer rate and solid content. Cov(X,Y) is the covariance of X and Y and is defined as $cov(X,Y)=E[(X-\mu_X)(Y-\mu_Y)]$, wherein $\mu_X$ and $\mu_Y$ are respectively the average of attribute X and the average of attribute Y.

Then, a significant coefficient between the process parameter and the critical quality attribute is acquired according to the correlation coefficient.

Herein, the critical process parameter having an influence on the product quality during actual production is screened out through the RSD analysis and the correlation analysis, data processing is carried out in an RSD analysis module and a correlation module of the PKS. Software SPSS and Minitab are recommended to be used for the purpose of more convenience.

For example, when Minitab is used for calculating the Pearson coefficient, an item parameter to be analyzed is selected in Minitab and a Pearson coefficient method is selected, and a P value displayed is a significant coefficient between a process parameter in accordance with the deviation threshold value and the critical quality attribute.

Finally, the critical process parameter is obtained by screening according to the significant coefficient.

During the correlation analysis, a parameter with the significant coefficient P value less than 0.05 is selected as CPP (or CPP is analyzed and selected according to actual conditions). Through the above two steps, CPPs corresponding to different CQAs of the work section can be obtained.

Through the method for screening CPP in this embodiment, the primary law between CQA and CPP in production is figured out to primarily deepen the understanding of the production process, and a good sample basis is laid for subsequent establishment of a mathematical model.

Wherein, a method for establishing the relationship model comprises the following steps:

First, data standardization is carried out on the critical process parameter and the critical quality attribute.

There are many standardization ways, such as standard deviation standardization and range standardization. All these standardization ways can be tried as many as possible in study to compare the differences of results.

Second, the standardized critical process parameter and critical quality attribute are fitted through a stepwise regression method to obtain the relationship model.

There are many regression ways such as linear regression and nonlinear regression. Stepwise regression is adopted herein (to avoid the influence of multicollinearity), and SVM, MLR, BP and the like can also be tried. In the embodiment of the present invention, Minitab is adopted for stepwise regression. Particularly, 'statistics'-'regression'-'fitting model' is selected in Minitab, a response variable and an independent variable are selected, select $\alpha$ and delete $\alpha$ are set in 'stepwise' option, and the mathematical model, namely a regression equation, is obtained after regression is ended.

Generally speaking, compared with laboratory data, a comprehensive evaluation method, such as a partial regression coefficient method or a multi-index comprehensive assessment method, will be adopted to screen CPPs in the case of many CQAs during small-scale research. However, due to the lack of experimental design during actual production, it is difficult to obtain a partial regression equation with high $R^2$ acquired by small-scale research, and thus, this method is inapplicable to actual production data. With the relative maturity of the production process, variable factors are generally several constant ones, and thus, the effect of screening CPPs from actual production data will be better by the combination of simple RSD and correlation analysis. But, the defects lie in that: due to the lack of a method for comprehensively evaluating and screening a multi-index critical process parameter, the situation of no interwork section of the design space may appear if CQAs are used for searching for the design space one by one, that is, compared with laboratory data, the determination coefficient $R^2$ in production is relatively small, so that a CPP screening method suitable for actual production is still needed (at present, CPPs corresponding to all CQAs are tried to be comprehensively used as an overall elevation index to expand the selection range of $\alpha$ during stepwise regression until $R^2$ is greater than 0.85).

Embodiment 4

In one embodiment of the present invention, a method for mining potential parameters of the design space is provided. Referring to FIG, 6, the method comprises the following steps:

S41: a request for mining potential parameters of the design space is received, wherein the request comprises work section condition information corresponding to the design space.

S42: a critical quality attribute corresponding to a work section is acquired according to the work section condition information and a potential parameter set is determined.

S43: determined potential parameters are tested to obtain to-be-verified potential parameters.

The specific testing process is preferably performed as follows: a single-factor test is carried out on the determined potential parameters to obtain multiple process parameters; and an orthogonal test is carried out on the process parameters to obtain the to-be-verified potential parameters.

Wherein, a method for acquiring the to-be-verified potential parameters comprises the following steps:

First, an orthogonal test is designed to obtain inter-group test data and intra-group test data.

Particularly, the inter-group test data are test data in different groups (for different process parameters), and the intra-group test data are test data in the same group (for the same process parameter).

Second, variance analysis is carried out on the inter-group test data and the intra-group test data to obtain a mean square-to-sum of deviation square ratio.

The process of variance analysis is as follows: a degree of freedom between groups and a degree of freedom in each group are determined according to the designed orthogonal test, and the sum of deviation square between the groups and the sum of deviation square in each group are calculated according to the inter-group test data and the intra-group test data; and then, the mean square between the groups is obtained by calculation according to the sum of deviation square between the groups and the degree of freedom between the groups, and the mean square in each group is obtained by calculation according to the sum of deviation square in each group and the degree of freedom in each group; and finally, the mean square-to-sum of deviation square ratio (F value) is obtained according to the mean square between groups and the mean square in each group.

Third, a corresponding significant coefficient is obtained according to the mean square-to-sum of deviation square ratio.

The matching significant coefficient P value corresponding to the F value can be determined by table look-up.

Fourth, the to-be-verified potential parameters are obtained by screening according to the significant coefficient.

A significant coefficient threshold is determined, and process parameters corresponding to significant coefficients smaller than the significant coefficient threshold are used as the to-be-verified potential parameters. That is, parameters with the significant coefficient P value less than 0.05 are used as the to-be-verified potential parameters (or the to-be-verified potential parameters are analyzed and selected according to actual conditions).

In addition to the above preferred method, the following mining method can also be adopted: a Plackett-Burman test is carried out on the determined potential process parameters, and process parameters are obtained by screening according to the screening rule. In this embodiment, the screening rule is that, the determined parameters with the significant coefficient less than or equal to 0.05 by the Plackett-Burman test are used as the screened process parameters.

Then, the following step is performed to obtain the to-be-verified potential parameters:

Relative standard deviation analysis is carried out on the process parameters to obtain process parameters in accordance with the deviation threshold; correlation analysis is carried out on the process parameters in accordance with the deviation threshold to obtain a correlation coefficient between the process parameters and the critical quality attribute; a significant coefficient between the process parameters and the critical quality attribute is obtained according to the correlation coefficient; and process parameters corresponding to significant coefficients smaller than the significant coefficient threshold are used as the to-be-verified potential process parameters.

S44: the to-be-verified potential parameters are verified to obtain the potential parameters of the design space;

Wherein, a method for verifying the to-be-verified parameters comprises the following steps:

First, the to-be-verified potential parameters are added into a critical process parameter library of the design space, and the critical process parameter set is updated.

As described in Embodiment 1, a potential parameter range is determined within a range beyond release parameters (namely CPPs), while the to-be-verified potential parameters are screened out from the potential parameter range. In this embodiment, the process of verifying the to-be-verified potential parameters is the process of optimizing the design space, that is, new CPPs formed by the to-be-verified potential parameters and the release parameters are used to search for a new design space.

Particularly, the updated design space can be verified to check whether or not the mined potential parameters have a beneficial effect on the production capability and performance of the PKS. The updated design space can be verified as follows: the process capability of the system before and after optimization is calculated; if the process capability is improved, it indicates that the potential parameters are successfully mined; or, if the process capability is reduced, the design space before optimization is recovered.

Embodiment 5

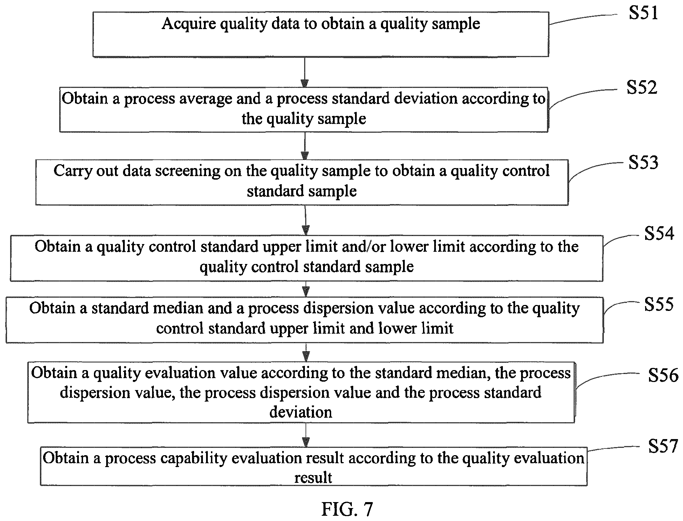

In this embodiment of the present invention, a method for evaluating the process capability of the system is provided. Referring to FIG. 7, the method comprises the following steps:

S51: quality data is acquired to obtain a quality sample, wherein the quality data is performance parameters of the intermediate in the production process;

S52: a process average value and a process standard deviation are obtained according to the quality sample;

S53: data screening is carried out on the quality sample to obtain a quality control standard sample;

For example, if the quality sample includes 100 pieces of quality data which are numbered 0-99, random sampling is carried out on the quality sample 100 (or 99, or 101 or other) times, one piece of quality data is randomly selected from the 0-99 pieces of quality data to serve as a member of a data set every time random sampling is carried out on the quality sample, the data set including 100 numbers will be obtained by 100 times of random sampling, and then the numbers in the data set are ranked from small to large. According to the screening rule (for example, 80% of data of the ranked sample is used as quality evaluation data of the work section), for the data set obtained after ranking from small to large, the 11th number is added into a first quality control standard sample, and the 90th number is added into a second quality control standard sample.

The above step is repeated, for example, 100,00 times, so that there are 100,00 quality control standard lower limits in a first quality control standard sample, and there are 100,00 quality control standard upper limits in a second quality control standard sample.

S54: a quality control standard upper limit and/or lower limit are/is obtained according to the quality control standard samples;

Particularly, sample averaging is carried out on the first quality control standard sample, that is, the 10,000 quality control standard lower limits in the first quality control standard sample are averaged to obtain the quality control standard lower limit; and sample averaging is carried out on the second quality control standard sample, that is, the 100,00 quality control standard upper limits in the second quality control standard sample are averaged to obtain the quality control standard upper limit.

S55: a standard median value and a process dispersion value are obtained according to the quality control standard upper limit and lower limit, wherein, standard median $\text{value} = \frac{\text{upper limit}+\text{lower limit}}{2}$ ,

$$\text{process dispersion value} = \frac{\text{upper limit}-\text{lower limit}}{2} ;$$

S56: a quality evaluation value is obtained according to the standard median value, the process dispersion value, the process average value and the process standard deviation by the following calculation formula;

$$\text{quality evaluation value} = \frac{\text{process dispersion value}- |\ \text{process average value}-\text{standard median value}|}{3*\text{process standard deviation}}$$

, wherein the process average value is an average of quality data of the quality sample, and the process standard deviation is a standard deviation of the quality data;

When the quality control standard only has an upper limit or a lower limit, a method for calculating the quality evaluation result comprises the following steps:

The quality evaluation value is obtained by the following calculation formula:

$$\text{quality evaluation value} = \frac{\min\ (\text{upper limit}-\text{process average value},\text{process average value}-\text{lower limit})}{3*\text{process standard deviation}}$$

, wherein, the process average value is an average of quality data of the quality sample, and the process standard deviation is a standard deviation of the quality data; and

S57: the process capability evaluation result is obtained according to a quality evaluation result.

Particularly, a corresponding capability evaluation mapping table is pre-established, wherein quality evaluation results and corresponding process capability evaluation results are set in the capability evaluation mapping table. For example, if the quality evaluation result is greater than or equal to 2.00, the process capability evaluation result is class A++; if the quality evaluation result is greater than or equal to 1.67 and is smaller than 2, the process capability evaluation result is class A+; if the quality evaluation result is greater than or equal to 1.33 and smaller than 1.67, the process capability evaluation result is class A; if the quality evaluation result is greater than or equal to 1.00 and smaller than 1.33, the process capability evaluation result is class B; if the quality evaluation result is greater than or equal to 0.67 and smaller than 1.00, the process capability evaluation result is class C; and if the quality evaluation result is smaller than 0.67, the process capability evaluation result is class D.

The process capability evaluation result can guide control over the production process. For example, if the process capability evaluation result is class A++, it indicates that the production capacity is excessive, and the cost should be reduced; if the process capability evaluation result is class A+, it indicates that the level can be maintained; if the process capability evaluation result is class A, it indicates that the process capability is good and can be properly improved; if the process capability evaluation result is class B, it indicates that the process capability is moderate, it can be properly improved in critical work sections and can be maintained in non-critical work sections; if the process capability evaluation result is class C, it indicates that the process capability needs to be improved; and if the process capability evaluation result is class D, it indicates that the process capability is severely insufficient, and a production halt for inspection is suggested.

In the case where there are multiple quality evaluation results, the process capability evaluation result is determined according to a capability evaluation rule. For example, the minimum one of the multiple quality evaluation results is selected, and a process capability evaluation result having a mapping relation with the minimum quality evaluation result is obtained to serve as a capability evaluation result of the multiple quality evaluation results. The capability evaluation rule of the present invention is not limited to the minimum quality evaluation result, and corresponding capability evaluation rule can be set according to the actual production condition.

Embodiment 6

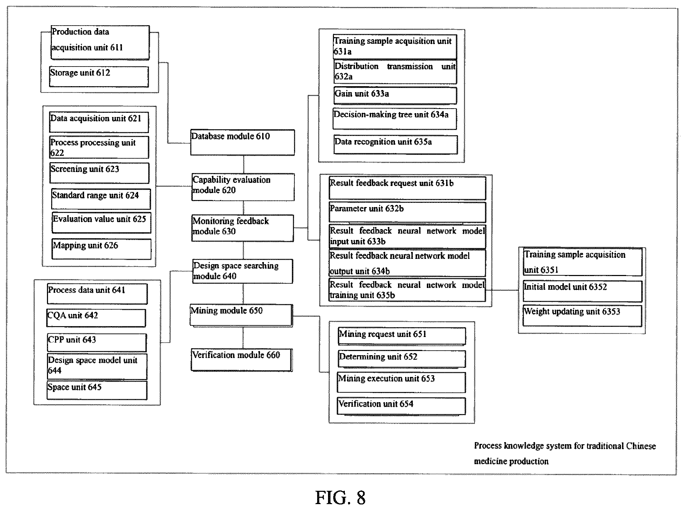

In one embodiment of the present invention, a process knowledge system for traditional Chinese medicine production is provided. As shown in FIG. 8, the system comprises:

A database module 610 comprising a production data acquisition unit 611 and a storage unit 612, wherein the production data acquisition unit is used for acquiring process parameter data in production, process parameter data comprises quality data and process data, and the storage unit is used for storing the acquired process parameter data;

A capability evaluation module 620 used for evaluating the process capability of a system according to the quality data to obtain a process capability evaluation result;

A monitoring feedback module 630 used for entering a whole-process monitoring mode in response to the process capability evaluation result indicating that the process capability is sufficient; and

A design space searching module 640 used for entering a design space searching mode in response to the process capability evaluation result indicating that the process capability is insufficient.

A large quantity of quality data and process parameter data used by the PKS is stored in the database module 610 and comprises univariate attributes such as the content, density and pH of index components, and spectrum and matrix data reflecting the overall attributes such as fingerprint, near infrared spectrum, and ultraviolet spectrum. A non-relational database characterized by being non-relational, distributed, source-opening and horizontally expandable is adopted.

Particularly, a Redis database is used for storing data, and the partition technology is adopted to fulfill the following advantages: 1, a larger database can be constructed by means of the sum of internal memories of multiple computers; 2, the calculation capability can be expanded by means of multiple multi-core computers, and the network bandwidth can be expanded by means of multiple computers and network adapters.

In the PKS, both the Redis database and the Hash partition technology are adopted to specifically implement the following steps:

Step 1: a Hash table is established, and a keyword in data is used as an index;

Step 2: a proper Hash function is selected; as for integer modulus, an integer is converted into a number from 0 to 3 so as to be mapped into one of the four Redis examples, for example, 93024922 % 4=2 refers to storage in example R2;

Step 3: if m and n separately represent the table length and node number filled in the table, $\alpha=n/m$ is defined as a load factor in the Hash table, wherein the greater $\alpha$ is, the fuller the table is, and the larger the probability of a confliction is, and generally, $\alpha 1$; when the load factor is greater than 0.75, the Hash table is subjected to Hash operation again to guarantee rapid storage of a large quantity of data, and a basis is laid for real-time data reading.

Data is read from the database module 610 in real time mainly through the following techniques:

1) Storage replica technique

The core scheme is that the disk array-to-disk array data block replica technique of a storage array is used to realize remote copy of quality data and process parameter data so as to realize disaster protection of the data. When a disaster happens to a main database center, an operation support environment can be created in a disaster recovery center by means of data in the disaster recovery center to support continuous operation of production. Meanwhile, a service system of the main database center can be recovered by means of data in the disaster recovery center, so that the normal operation state before the disaster can be rapidly recovered.

2) ETL technique

ETL is a data extract, transform and load process and provides a method for transforming data from a source system to a target system. Particularly, different data extract, transform and load programs are compiled for different quality data and process parameter data to complete most data integration work.

Wherein, the main functional technique is a clean data transform function, namely, field mapping, automatic matching of mapping, field splitting, hybrid operation of multiple fields, correlation of trans-heterogeneous databases, self-defining functions, multi-data type support, complex condition filter, dirty read support, batch load of data, transform of time type, support of various code tables, dynamic modification of environment variables, de-duplication record, breakpoint extract, record combination or calculation, record splitting, whether or not extracted fields can be dynamically modified, common transform functions such as line/column transform, ranking, statistics, and weights and measures, generation and debugging of surrogate main keys, extract of remote data, processing of increment extract, production sample data, whether or not the data comparison function is supported in the transform process, data preview, performance monitoring, date cleaning and standardization, and line/column grouping and combination.

By adoption of the above techniques in the PKS, it is guaranteed to the maximum extent that data can be rapidly and concurrently read in real time, and a basis is laid for subsequent data analysis and feedback.

Particularly, the design space searching module 640 comprises:

A process data unit 641 used for acquiring process data, wherein the process data comprises quality parameters of an intermediate obtained in a previous work section;

A CQA unit 642 used for selecting a type of a critical quality attribute according to work section production conditions;

A CPP unit 643 used for screening out process data relating to the critical quality attribute so as to use the screened-out process data as a critical process parameter;

A design space model unit 644 used for establishing a relationship model between the critical process parameter and the critical quality attribute; and

A space unit 645 used for acquiring a design space according to the relationship model, wherein the design space is a specific range corresponding to the critical quality attribute.

The process knowledge system for traditional Chinese medicine production in the embodiment further comprises a mining module 650, and the mining module 650 comprises:

A mining request unit 651 used for receiving a request for mining potential parameters of the design space, wherein the request comprises work section condition information corresponding to the design space;

A determining unit 652 used for acquiring the critical quality attribute corresponding to a work section according to the work section condition information and determining a potential parameter set;

A mining execution unit 653 used for testing determined potential parameters to obtain to-be-verified potential parameters; and

A verification unit 654 used for verifying the to-be-verified potential parameters to obtain the potential parameters of the design space.

Preferably, the capability evaluation module 620 comprises:

A data acquisition unit 621 used for acquiring quality data from the database module 610 to obtain a quality sample, wherein the quality data is performance parameters of the intermediate in the production process;

A process processing unit 622 used for obtaining a process average value and a process standard deviation according to the quality sample;

A screening unit 623 used for carrying out data screening on the quality sample to obtain a quality control standard sample;

A standard range unit 624 used for obtaining a quality control standard upper limit and/or lower limit according to the quality control standard sample;

An evaluation value unit 625 used for obtaining a standard median value and a process dispersion value according to the quality control standard upper limit and lower limit, wherein,

$$\text{standard median value} = \frac{\text{upper limit} + \text{lower limit}}{2}$$

$$, \text{process dispersion value} = \frac{\text{upper limit} - \text{lower limit}}{2} ;$$

and

obtaining a quality evaluation value according to the standard median value, the process dispersion value, the process average value and the process standard deviation by the following calculation formula;

$$\text{quality evaluation value} = \frac{\text{process dispersion value} - |\text{ process average value} - \text{standard median value}|}{3 * \text{process standard deviation}}$$

, wherein the process average value is an average of quality data of the quality sample, and the process standard deviation is a standard deviation of the quality data; and

A mapping unit 626 used for obtaining the process capability evaluation result according to the quality evaluation result.

In one embodiment of the present invention, the monitoring feedback module 630 is used for multi-dimensional data recognition and comprises:

A training sample acquisition unit 631a used for acquiring multiple training samples to form a training sample set, wherein each training sample comprises multiple process parameters, each process parameter has a corresponding attribute parameter and a corresponding attribute category, and there are multiple combinations of the attribute parameters and categories;

A distribution transmission unit 632a used for acquiring a distribution transmission information value of the training sample set according to the categories of the training sample set;

A gain unit 633a used for acquiring an information gain of each process parameter according to the distribution transmission information value;

A decision tree unit 634a used for selecting the process parameter with the maximum information gain as a split node to establish a decision tree; and

A data recognition unit 635a used for carrying out category recognition on new data according to the decision tree.

In another embodiment of the present invention, the monitoring feedback module 630 is used for carrying out result feedback based on process parameters and comprises:

A result feedback request unit 631b used for receiving a result feedback request which comprises an intermediate result type;

A parameter unit 632b used for acquiring process parameters corresponding to the feedback request to form a process parameter set, wherein the process parameters are multi-dimensional parameters;

A result feedback neural network model input unit 633b used for inputting the process parameter set to a result feedback neural network model, wherein the result feedback neural network model is obtained by training with process parameter samples; and

A result feedback neural network model output unit 634b used for acquiring an output result of the result feedback neural network model.

Wherein, the monitoring feedback module 630 further comprises a result feedback neural network model training unit 635b which comprises:

A training sample acquisition unit 6351 used for acquiring data of process parameter samples to be trained, wherein the process parameter samples include multiple process parameter sets and corresponding given target values;

An initial model unit 6352 used for establishing an initial network model which includes an input layer, a hidden layer, an output layer, an initial weight and an initial offset; and

A weight updating unit 6353 used for updating the initial weight and the initial offset through a back-propagation method until weight convergence is realized, so as to obtain the result feedback neural network model.

It should be noted that: when the process knowledge system for traditional Chinese medicine production is used for control over a production process, the partition of the above-mentioned functional modules is only for the purpose of illustrative explanation. In actual application, the above functions can be completed by different functional modules as needed, that is, the internal structure of the knowledge system for traditional Chinese medicine production can be divided into different functional modules to complete part or all of the functions described above. In addition, the embodiment of the knowledge system for traditional Chinese medicine production and the process control method for traditional Chinese medicine production in the above embodiment are based on the same concept, can be specifically implemented as the embodiment of the method, and will not be described anymore herein.

The above embodiments are only preferred ones of the present invention, and are not intended to limit the present invention. Any modifications, equivalent substitutions and improvements made on the basis of the spirit and principle of the present invention should fall within the protection scope of the present invention.

**Claims**

1. A process control method for traditional Chinese medicine production, comprising:

   acquiring process parameter data in production, wherein the process parameter data comprises quality data and process data;
   evaluating a process capability of a system to obtain a process capability evaluation result according to the quality data;
   entering a whole-process monitoring mode if the process capability evaluation result is sufficient;
   entering a design space searching mode according to the process data if the process capability evaluation result is insufficient.

2. The method according to Claim 1, wherein the entering a design space searching mode comprises:

   acquiring the process data, wherein the process data comprises quality parameters of an intermediate obtained in a previous work section;
   selecting a type of a critical quality attribute according to work section production conditions;
   screening out process data relating to the critical quality attribute, and using the screened-out process data as a critical process parameter;
   establishing a relationship model between the critical process parameter and the critical quality attribute; and
   acquiring a design space according to the relationship model, wherein the design space is a specific range corresponding to the critical quality attribute.

3. The method according to Claim 1, wherein after the entering a design space searching mode, the method further comprises:

   releasing parameters according to the acquired design space;
   re-evaluating the process capability of the system to obtain a process capability re-evaluation result;
   entering the whole-process monitoring mode if the process capability re-evaluation result is sufficient;
   mining potential parameters of the design space if the process capability re-evaluation result is insufficient.

4. The method according to Claim 3, wherein the mining potential parameters of the design space comprises:

   receiving a request for mining potential parameters of the design space, wherein the request comprises work section condition information corresponding to the design space;

acquiring a critical quality attribute corresponding to a work section and determining a potential parameter set, according to the work section condition information;

testing determined potential parameters to obtain to-be-verified potential parameters; and

verifying the to-be-verified potential parameters to obtain the potential parameters of the design space.

5. The method according to Claim 1, wherein the evaluating a process capability of a system to obtain a process capability evaluation result comprises:

acquiring quality data to obtain a quality sample, wherein the quality data is performance parameters of an intermediate in a production process;

obtaining a process average value and a process standard deviation according to the quality sample;

carrying out data screening on the quality sample to obtain a quality control standard sample;

obtaining a quality control standard upper limit and/or lower limit according to the quality control standard sample;

obtaining a standard median value and a process dispersion value according to the quality control standard upper limit and lower limit, wherein,

$$\text{standard median value} = \frac{\text{upper limit} + \text{lower limit}}{2}$$

$$\text{process dispersion value} = \frac{\text{upper limit} - \text{lower limit}}{2};$$

obtaining a quality evaluation value according to the standard median value, the process dispersion value, the process average value and the process standard deviation by the following calculation formula,

$$\text{quality evaluation value} = \frac{\text{process dispersion value} - |\text{process average value} - \text{standard median value}|}{3 * \text{process standard deviation}}$$

wherein the process average value is an average of quality data of the quality sample, and the process standard deviation is a standard deviation of the quality data; and

obtaining the process capability evaluation result according to the quality evaluation result.

6. The method according to Claim 1, wherein the entering a whole-process monitoring mode comprises multi-parameter recognition which comprises:

acquiring multiple training samples to form a training sample set, wherein each said training sample comprises multiple process parameters, each said process parameter comprises a corresponding attribute parameter and a corresponding class, and there are multiple combinations of the attribute parameters and classes;

acquiring a distribution transmission information value of the training sample set according to the classes in the training sample set;

acquiring an information gain of each said process parameter according to the distribution transmission information value;

selecting the process parameter with a maximum information gain as a split node to establish a decision tree; and

carrying out class recognition on new data according to the decision tree.

7. The method according to Claim 1, wherein the entering a whole-process monitoring mode comprises process parameter-based result feedback which comprises:

receiving a result feedback request, wherein the result feedback request comprises an intermediate result type;

acquiring process parameters corresponding to the feedback request to form a process parameter set, wherein the process parameters are multi-dimensional parameters; and

inputting the process parameter set to a result feedback neural network model, wherein the result feedback neural network model is obtained by training with process parameter samples; and

acquiring an output result of the result feedback neural network model.

8. The method according to Claim 7, wherein the result feedback neural network model is obtained by training with the process parameter samples via the following steps:

acquiring data of process parameter samples to be trained, wherein the process parameter samples comprise multiple process parameter sets and corresponding given target values;
establishing an initial network model, wherein the initial network model comprises an input layer, a hidden layer, an output layer, an initial weight and an initial offset; and
updating the initial weight and the initial offset through a back-propagation method until weight convergence is realized, so as to obtain the result feedback neural network model.

9. A process knowledge system for traditional Chinese medicine production, comprising:

a database module, comprising a production data acquisition unit and a storage unit, wherein the production data acquisition unit is used for acquiring process parameter data in production, the parameter data comprises quality data and process data, and the storage unit is used for storing the acquired process parameter data;
a capability evaluation module, used for evaluating a process capability of a system to obtain a process capability evaluation result according to the quality data;
a monitoring feedback module, used for entering a whole-process monitoring mode in response to the process capability evaluation result is sufficient; and
a design space searching module, used for entering a design space searching mode in response to the process capability evaluation result is insufficient.

10. The system according to Claim 9, wherein the design space searching module comprises:

a process data unit, used for acquiring the process data, wherein the process data comprises quality parameters of an intermediate obtained in a previous work section;
a CQA unit, used for selecting a type of a critical quality attribute according to work section production conditions;
a CPP unit, used for screening out process data relating to the critical quality attribute to use the screened-out process data as a critical process parameter;
a design space model unit, used for establishing a relationship model between the critical process parameter and the critical quality attribute; and
a space unit, used for acquiring a design space according to the relationship model, wherein the design space is a specific range corresponding to the critical quality attribute.

11. The system according to Claim 9, wherein the system further comprises a mining module which comprises:

a mining request unit, used for receiving a request for mining potential parameters of a design space, wherein the request comprises work section condition information corresponding to the design space;
a determining unit, used for acquiring a critical quality attribute corresponding to a work section and determining a potential parameter set according to the work section condition information;
a mining execution unit, used for testing determined potential parameters to obtain to-be-verified potential parameters; and
a verification unit, used for verifying the to-be-verified potential parameters to obtain the potential parameters of the design space.

12. The system according to Claim 9, wherein the capability evaluation module comprises:

a data acquisition unit, used for acquiring quality data to obtain a quality sample, wherein the quality data are performance parameters of an intermediate in a production process;
a process processing unit, used for obtaining a process average value and a process standard deviation according to the quality sample;
a screening unit, used for carrying out data screening on the quality sample to obtain a quality control standard sample;
a standard range unit, used for obtaining a quality control standard upper limit and/or lower limit according to the quality control standard sample;
an evaluation value unit, used for obtaining a standard median value and a process dispersion value according to the quality control standard upper limit and lower limit, wherein,

$$\text{standard median value} = \frac{\text{upper limit} + \text{lower limit}}{2}$$

$$, \text{process dispersion value} = \frac{\text{upper limit} - \text{lower limit}}{2};$$

and

obtaining a quality evaluation value according to the standard median value, the process dispersion value, the process average value and the process standard deviation by the following calculation formula;

$$\text{quality evaluation value} = \frac{\text{process dispersion value} - |\text{process average value} - \text{standard median value}|}{3 * \text{process standard deviation}}$$

, wherein the process average value is an average of quality data of the quality sample, and the process standard deviation is a standard deviation of the quality data; and

a mapping unit, used for obtaining the process capability evaluation result according to the quality evaluation result.

13. The system according to Claim 9, wherein the monitoring feedback module is used for multi-dimensional parameter recognition and comprises:

a training sample acquisition unit, used for acquiring multiple training samples to form a training sample set, wherein each said training sample comprises multiple process parameters, each said process parameter comprises a corresponding attribute parameter and a corresponding class, and there are multiple combinations of the attribute parameters and classes;

a distribution transmission unit, used for acquiring a distribution transmission information value of the training sample set according to the classes in the training sample set;

a gain unit, used for acquiring an information gain of each said process parameter according to the distribution transmission information value;

a decision tree unit, used for selecting the process parameter with a maximum information gain as a split node to establish a decision tree; and

a data recognition unit, used for carrying out class recognition on new data according to the decision tree.

14. The system according to Claim 9, wherein the monitoring feedback module is used for carrying out result feedback based on process parameters and comprises:

a result feedback request unit, used for receiving a result feedback request which comprises an intermediate result type;

a parameter unit, used for acquiring process parameters corresponding to the feedback request to form a process parameter set, wherein the process parameters are multi-dimensional parameters;

a result feedback neural network model input unit, used for inputting the process parameter set to a result feedback neural network model obtained by training process parameter samples; and

a result feedback neural network model output unit, used for acquiring an output result of the result feedback neural network model.

15. The system according to Claim 14, wherein the monitoring feedback module further comprises a result feedback neural network model training unit which comprises:

a training sample acquisition unit, used for acquiring data of process parameter samples to be trained, wherein the process parameter samples comprise multiple process parameter sets and corresponding given target values;

an initial model unit, used for establishing an initial network model which comprises an input layer, a hidden layer, an output layer, an initial weight and an initial offset; and

a weight updating unit, used for updating the initial weight and the initial offset through a back-propagation method until weight convergence is realized, so as to obtain the result feedback neural network model.

Acquire process parameter data in production ———————— S1

Evaluate the process capability of a system according to the quality data to obtain a process capability evaluation result ———————— S2

Whether or not the process capability is sufficient? —— Y —— Enter a whole-process monitoring mode ———— S3

N

Enter a design space searching mode according to process data ———————— S4

FIG. 1

Data collection → Quality data → Primary evaluation of the process capability —— Sufficient → Whole-process monitoring → Intelligent feedback

—Insufficient—

Process data → Dominant CPP → Screen CPP → Regression analysis

—Add—

Potential CPP

Design space

Laboratory research ←—Insufficient—— Re-evaluation of the process capability —Sufficient→ Whole-process monitoring → Intelligent feedback

FIG. 2

Up to the cycle time? — Yes — Yes — Up to the cycle time?

Acquire process parameter data in production — S21

Evaluate the process capability of a system according to the quality data to obtain a process capability evaluation result — S22

Whether or not the process capability is sufficient? — Y — Enter a whole-process monitoring mode — S23

N

S24 — Enter a design space searching mode according to process data

A1 — Acquire multiple training samples to form a training sample set

B1 — Receive a result feedback request

S241 — Release parameters according to an acquired design space

A2 — Acquire a distribution transmission information value of the training sample set

B2 — Acquire process parameters corresponding to the feedback request

S242 — Re-evaluate the process capability of the system to obtain a process capability re-evaluation result

A3 — Acquire an information gain of each process parameter

B3 — Input a process parameter set to a result feedback neural network model

S243 — Enter a whole-process monitoring mode — Y — Whether or not the process capability is sufficient?

A4 — Select the process parameter with the maximum information gain as a split node to establish a decision-making tree

B4 — Acquire an output result of the result feedback neural network model

N

S244 — Mine potential parameters of the design space

A5 — Carry out class recognition on new data according to the decision-making tree

S245 — Add the mined potential parameters into a release parameter set to optimize the design space

FIG. 3

Acquire data of process parameter samples to be trained — B31

Establish an initial network model — B32

Update the initial weight and the initial offset through a back-propagation method until weight convergence is realized, so as to obtain the result feedback neural network model — B33

FIG. 4

Acquire process data — S31

Select the type of a critical quality attribute according to work section production conditions — S32

Screen out process data relating to the critical quality attribute, and use the screened out process data as a critical process parameter — S33

Establish a relationship model between the critical process parameter and the critical quality attribute — S34

Acquire a design space according to the relationship model — S35

FIG. 5

Receive a request for mining potential parameters of the design space — S41

Acquire a critical quality attribute corresponding to a work section and determine a potential parameter set — S42

Test determined potential parameters to obtain to-be-verified potential parameters — S43

Verify the to-be-verified potential parameters to obtain potential parameters of the design space — S44

FIG. 6

Acquire quality data to obtain a quality sample ⟶ S51

Obtain a process average and a process standard deviation according to the quality sample ⟶ S52

Carry out data screening on the quality sample to obtain a quality control standard sample ⟶ S53

Obtain a quality control standard upper limit and/or lower limit according to the quality control standard sample ⟶ S54

Obtain a standard median and a process dispersion value according to the quality control standard upper limit and lower limit ⟶ S55

Obtain a quality evaluation value according to the standard median, the process dispersion value, the process dispersion value and the process standard deviation ⟶ S56

Obtain a process capability evaluation result according to the quality evaluation result ⟶ S57

FIG. 7

FIG. 8

FIG. 9

FIG. 10

Weight

$y_1$ $w_{1j}$

$y_2$ $w_{2j}$

$\vdots$

$y_n$ $w_{nj}$

Offset

$\theta_j$

$\sum$

$f$ → Output

Input
(output of a previous layer)

Weight sum

Activation function

FIG. 11

**INTERNATIONAL SEARCH REPORT**

| International application No. |
| --- |
| **PCT/CN2018/099310** |

**A. CLASSIFICATION OF SUBJECT MATTER**

G06N 5/02(2006.01)i

According to International Patent Classification (IPC) or to both national classification and IPC

**B. FIELDS SEARCHED**

Minimum documentation searched (classification system followed by classification symbols)

G06N5/-

Documentation searched other than minimum documentation to the extent that such documents are included in the fields searched

Electronic data base consulted during the international search (name of data base and, where practicable, search terms used)

USTXT; EPTXT; CNTXT; CNABS; WOTXT; VEN; CNKI: 中药, 知识库, 采集, 模型, 评价, 筛选, 监控, 数据, 质量, 参数, 样本, 决策树, 神经网络, 反馈, Chinese medicine, knowledge system, collect, model, evaluation, filter, monitor, data, quality, parameter, sample, decision tree, neural network, feedback

**C. DOCUMENTS CONSIDERED TO BE RELEVANT**

| Category* | Citation of document, with indication, where appropriate, of the relevant passages | Relevant to claim No. |
| --- | --- | --- |
| PX | CN 107578104 A (JIANGSU KANION PHARMACEUTICAL CO., LTD. ET AL.) 12 January 2018 (2018-01-12)<br>entire document | 1-15 |
| X | CN 105867129 A (SUZHOU INDUSTRIAL TECHNOLOGY RESEARCH INSTITUTE OF ZHEJIANG, UNIVERSITY) 17 August 2016 (2016-08-17)<br>description, paragraphs 57-69 | 1-15 |
| A | CN 104050612 A (WUXI XIANGXIANG BIOTECHNOLOGY CO., LTD.) 17 September 2014 (2014-09-17)<br>entire document | 1-15 |
| A | CN 104833651 A (ZHEJIANG UNIVERSITY ET AL.) 12 August 2015 (2015-08-12)<br>entire document | 1-15 |
| A | CN 104865322 A (ZHEJIANG UNIVERSITY ET AL.) 26 August 2015 (2015-08-26)<br>entire document | 1-15 |
| A | US 2015356448 A1 (BATTELLE MEMORIAL INSTITUTE) 10 December 2015 (2015-12-10)<br>entire document | 1-15 |

☐ Further documents are listed in the continuation of Box C.　　☑ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **12 October 2018** | **13 November 2018** |

| Name and mailing address of the ISA/CN | Authorized officer |
| --- | --- |
| **State Intellectual Property Office of the P. R. China**<br>**No. 6, Xitucheng Road, Jimenqiao Haidian District, Beijing**<br>**100088**<br>**China** | |
| Facsimile No. **(86-10)62019451** | Telephone No. |

Form PCT/ISA/210 (second sheet) (January 2015)

**INTERNATIONAL SEARCH REPORT**
Information on patent family members

| International application No. |
|---|
| **PCT/CN2018/099310** |

| Patent document cited in search report | | | Publication date (day/month/year) | Patent family member(s) | | | Publication date (day/month/year) |
|---|---|---|---|---|---|---|---|
| CN | 107578104 | A | 12 January 2018 | None | | | |
| CN | 105867129 | A | 17 August 2016 | None | | | |
| CN | 104050612 | A | 17 September 2014 | None | | | |
| CN | 104833651 | A | 12 August 2015 | CN | 104833651 | B | 30 June 2017 |
| CN | 104865322 | A | 26 August 2015 | None | | | |
| US | 2015356448 | A1 | 10 December 2015 | US | 2012239605 | A1 | 20 September 2012 |
| | | | | US | 2014180997 | A1 | 26 June 2014 |
| | | | | US | 9111216 | B2 | 18 August 2015 |
| | | | | US | 8666922 | B2 | 04 March 2014 |
| | | | | US | 9424522 | B2 | 23 August 2016 |
| | | | | US | 2016371592 | A1 | 22 December 2016 |